# EUROPÄISCHE PATENTANMELDUNG

(11) **EP 2 929 869 A1**
(43) Veröffentlichungstag der Anmeldung: **14.10.2015**
(21) Anmeldenummer: 15162826.0
(22) Anmeldetag: 08.04.2015
(51) Int. Cl.: A61J 3/00, B01F 13/10, A61J 7/00

(54) **Vorrichtung zu einer Herstellung und/oder zu einer Verabreichung**

(30) Priorität: 09.04.2014 DE 102014105058
(71) Anmelder: Ittstein, Stephanie, 8268 Salenstein (CH)
(72) Erfinder: Ittstein, Stephanie, 8268 Salenstein (CH)
(74) Vertreter: Daub, Thomas

(57) **Zusammenfassung**

Es wird eine Vorrichtung zu einer Herstellung von zumindest einer individuell angepassten Verabreichungs- und/oder Betriebshilfsstoffeinheit und/oder zu einer Verabreichung von zumindest einer individuell angepassten Verabreichungseinheit in Abhängigkeit von zumindest einer individuellen Kenngröße vorgeschlagen.

## Beschreibung

### Stand der Technik

Die Erfindung betrifft eine Vorrichtung zu einer Herstellung von zumindest einer Verabreichungs- und/oder Betriebshilfsstoffeinheit und/oder zu einer Verabreichung von zumindest einer Verabreichungseinheit.

Zudem betrifft die Erfindung ein Verfahren zu einem Betrieb der Vorrichtung.

In vielen Bereichen des täglichen Lebens werden Verabreichungs- und/oder Betriebshilfsstoffeinheiten in nicht individuell ermittelter Rezeptur und/oder Dosierung hergestellt und/oder verabreicht. Hierdurch kann es zu einer Unter- oder Überdosierung von Verabreichungs- und/oder Betriebshilfsstoffeinheiten kommen. Dies kann zu einem unzureichenden Nutzprofil der Verabreichungs- und/oder Betriebshilfsstoffeinheiten führen, welches zu Risiken führen kann.

Die Aufgabe der Erfindung besteht hierbei insbesondere darin, eine Vorrichtung zur Verfügung zu stellen, die eine Verabreichung von zumindest einer auf individuelle Bedürfnisse angepasste Zusammensetzung aufweisende Verabreichungseinheit ermöglicht und/oder die eine Herstellung von zumindest einer Verabreichungs- und/oder Betriebshilfsstoffeinheit ermöglicht, die eine auf individuelle Bedürfnisse angepasste Zusammensetzung aufweist. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Es wird eine Vorrichtung zu einer Herstellung von zumindest einer individuell angepassten Verabreichungs- und/oder Betriebshilfsstoffeinheit und/oder zu einer Verabreichung von zumindest einer individuell angepassten Verabreichungseinheit in Abhängigkeit von zumindest einer individuellen Kenngröße vorgeschlagen.

Der Ausdruck "individuell angepasst" soll hierbei insbesondere eine Zusammensetzung der Verabreichungs- und/oder Betriebshilfsstoffeinheit definieren, die speziell auf ein Individuum und/oder eine Anwendung abgestimmt ist. Somit weist die mittels der erfindungsgemäßen Vorrichtung hergestellte Verabreichungs- und/oder Betriebshilfsstoffeinheit vorzugsweise eine Zusammensetzung auf, bei der ein Zustand eines Individuums und/oder eine Art einer Anwendung während einer Herstellung und/oder einer Verabreichung berücksichtigbar ist.

Der Begriff "Verabreichungs- und/oder Betriebshilfsstoffeinheit" soll hier insbesondere eine Einheit definieren, die von einem Individuum verabreichbar ist, auf einer Haut eines Individuums oder eines Tiers applizierbar ist oder als Arbeitsmittel (Betriebsstoff oder Hilfsstoff) ausgebildet ist. Die mittels der Vorrichtung herstellbare Verabreichungs- und/oder Betriebshilfsstoffeinheit ist flüssig, gasförmig oder fest ausbildbar. Somit ist die Verabreichungs- und/oder Betriebshilfsstoffeinheit als Tablette, als Flüssigkeit, als Paste, als Creme, als Pulver o. dgl. ausbildbar. Somit kann die Verabreichungs- und/oder Betriebshilfsstoffeinheit als Tablette, als Getränk, als Creme, als Paste, als Flüssigkeit als Gas o. dgl. ausgebildet sein. Die Verabreichungs- und/oder Betriebshilfsstoffeinheit kann hierbei als Nahrungsmittel, als Nahrungsergänzungsmittel, als Medikament, als Kosmetika, als Genussmittel, als Futtermittel, als Schmiermittel o. dgl. ausgebildet sein.

Unter einer "individuellen Kenngröße" soll hier insbesondere eine Kenngröße verstanden werden, die einen Zustand eines Individuums und/oder eine Art einer Nutzung beschreibt und/oder definiert. Besonders bevorzugt ist die individuelle Kenngröße zu einer Herstellung und/oder Verabreichung der Verabreichungs- und/oder Betriebshilfsstoffeinheit als Vitalkenngröße eines Individuums, als Gesundheitszustandskenngröße eines Individuums, als Leistungskenngröße eines Individuums, als Anwendungskenngröße o. dgl. ausgebildet. Somit ist die zumindest eine individuelle Kenngröße beispielsweise als Puls, als Blutdruck, als Herzrhythmus, als Blutbild, als Zahnzustandskenngröße, als Hautverträglichkeitskenngröße, als Leistungs-EKG-Kenngröße, als Schmierstoffviskosität, als Bauteilleimartverbindungskenngröße o. dgl. ausgebildet.

Die Vorrichtung ist als Endverbrauchervorrichtung, als Industrievorrichtung, als Handwerkervorrichtung o. dgl. ausbildbar. Besonders bevorzugt ist die Vorrichtung als Endverbrauchervorrichtung ausgebildet. Somit ist die Vorrichtung vorzugsweise von einem Endverbraucher erwerbbar und/oder nutzbar. Die Vorrichtung ist besonders bevorzugt zu einer Herstellung von zumindest einer individuell angepassten Verabreichungs- und/oder Betriebshilfsstoffeinheit in Abhängigkeit von der zumindest einen individuellen Kenngröße und zu einer Verabreichung von zumindest einer individuell angepassten Verabreichungseinheit in Abhängigkeit von der zumindest einen individuellen Kenngröße vorgesehen. Unter "vorgesehen" soll insbesondere speziell ausgelegt und/oder speziell ausgestattet verstanden werden. Darunter, dass ein Element und/oder eine Einheit zu einer bestimmten Funktion vorgesehen ist, soll insbesondere verstanden werden, dass das Element und/oder die Einheit diese bestimmte Funktion in zumindest einem Anwendungs- und/oder Betriebszustand erfüllen/erfüllt und/oder ausführen/ausführt. Die Vorrichtung ist hierbei insbesondere dazu vorgesehen, Bestandteile der Verabreichungs- und/oder Betriebshilfsstoffeinheit in Abhängigkeit von der zumindest einen individuellen Kenngröße miteinander zu vermengen. Somit ist eine individuelle Konzentration von einzelnen Bestandteilen der Verabreichungs- und/oder Betriebshilfsstoffeinheit abhängig von der zumindest einen individuellen Kenngröße herstellbar. Eine Herstellung der individuell angepassten Verabreichungs- und/oder Betriebshilfsstoffeinheit erfolgt hierbei anhand einer individuell generierten Rezeptur, die abhängig von der zumindest einen individuellen Kenngröße ist. Die erfindungsgemäße Vorrichtung ist vorteilhaft zu einer Notfallmedikation, zu einer Herstellung von einer individuell angepassten Babynahrung, zu einer Herstellung von einer individuell angepassten Creme, zu einer Herstellung von einer individuell angepassten Impfungssubstanz, zu einer Herstellung von individuell angepassten Nahrungsmitteln o. dgl. nutzbar.

Zudem ist die Vorrichtung zusätzlich zu einer Herstellung der Verabreichungs- und/oder Betriebshilfsstoffeinheit dazu vorgesehen, eine Menge der mittels der Vorrichtung hergestellten Verabreichungs- und/oder Betriebshilfsstoffeinheit in Abhängigkeit von der zumindest einen individuellen Kenngröße zumindest teilweise automatisch zu verabreichen, insbesondere einem Menschen oder einem Tier zumindest teilweise automatisch zu verabreichen. In einer alternativen Ausgestaltung ist die Vorrichtung lediglich zu einer Herstellung der individuell angepassten Verabreichungs- und/oder Betriebshilfsstoffeinheit in Abhängigkeit von zumindest einer individuellen Kenngröße vorgesehen. In einer weiteren alternativen Ausgestaltung ist die Vorrichtung insbesondere lediglich zu einer Verabreichung von zumindest einer individuell angepassten Verabreichungseinheit in Abhängigkeit von zumindest einer individuellen Kenngröße vorgesehen.

Mittels der erfindungsgemäßen Vorrichtung kann vorteilhaft eine Verabreichungs- und/oder Betriebshilfsstoffeinheit hergestellt und/oder verabreicht werden, die an spezielle Bedürfnisse angepasst ist. Somit kann vorteilhaft eine Unter- oder Überdosierung der Verabreichungs- und/oder Betriebshilfsstoffeinheit vermieden werden. Somit kann ein vorteilhaftes Nutzprofil der Verabreichungs- und/oder Betriebshilfsstoffeinheit ermöglicht werden.

Vorteilhafterweise umfasst die Vorrichtung zumindest eine Rezeptur- und/oder Dosierungsgenerierungseinheit, die dazu vorgesehen ist, eine Rezeptur und/oder eine Dosierung in Abhängigkeit von der zumindest einen individuellen Kenngröße zu generieren. Die Rezeptur- und/oder Dosierungsgenerierungseinheit ist vorzugsweise dazu vorgesehen, eine Rezeptur zur Herstellung der Verabreichungs- und/oder Betriebshilfsstoffeinheit in Abhängigkeit von der zumindest einen individuellen Kenngröße zu generieren und/oder eine Dosierung der Verabreichungs- und/oder Betriebshilfsstoffeinheit in Abhängigkeit von der zumindest einen individuellen Kenngröße vorzunehmen. Somit kann vorteilhaft eine Rezeptur generiert werden und/oder eine Dosierung vorgenommen werden, die speziell auf einen Zustand eines Individuums und/oder auf eine Anwendungsart der Verabreichungs- und/oder Betriebshilfsstoffeinheit angepasst ist.

Zudem wird vorgeschlagen, dass die Rezeptur- und/oder Dosierungsgenerierungseinheit zumindest eine Steuer- und/oder Regeleinheit umfasst, die zumindest ein Betriebsprogramm aufweist, das in Abhängigkeit einer Eingabe von der zumindest einen individuellen Kenngröße ein Rezeptur- und/oder Dosierungsprofil erstellt. Unter einer "Steuer- und/oder Regeleinheit" soll insbesondere eine Einheit mit zumindest einer Steuerelektronik verstanden werden. Unter einer "Steuerelektronik" soll insbesondere eine Einheit mit einer Prozessoreinheit und mit einer Speichereinheit sowie mit einem in der Speichereinheit gespeicherten Betriebsprogramm verstanden werden. Das Rezeptur- und/oder Dosierungsprofil ist insbesondere derart ausgelegt, dass bei einer Eingabe zumindest einer weiteren individuellen Kenngröße zu einem späteren Zeitpunkt auf bereits eingegebenen Daten des Rezeptur- und/oder Dosierungsprofils zurückgegriffen wird und diese bei einer Generierung einer Rezeptur und/oder einer Dosierung berücksichtigt werden. Hierbei ist das Betriebsprogramm der Steuer- und/oder Regeleinheit vorzugsweise als Analyse-Betriebsprogramm ausgebildet. Hierbei umfasst das Betriebsprogramm vorzugsweise ein Abfrageteilprogramm, das gezielt individuelle Kenngrößen und/oder Daten abfragt, die zu einer Generierung einer individuell angepassten Rezeptur und/oder Dosierung notwendig sind. Mittels der erfindungsgemäßen Ausgestaltung kann vorteilhaft eine einfache Bedienbarkeit ermöglicht werden, da eine erneute Eingabe von allen erforderlichen Daten bei einer Änderung einer einzelnen Kenngröße vermeidbar ist. Infolge von regelmäßigen Eingaben von individuellen Kenngrößen ist das Rezeptur- und/oder Dosierungsprofil vorteilhaft an neue Gegebenheiten anpassbar. Hierbei ist es denkbar, dass infolge einer Eingabe und/oder einer automatischen Erfassung der zumindest einen individuellen Kenngröße, wie beispielsweise Alter, Größe, Gewicht, Ernährungs- und/oder Bewegungsverhalten o. dgl., mittels des Betriebsprogramms ein interaktiver Check-Up erfolgt und eine erforderliche Nährstoffmenge der herzustellenden Verabreichungseinheit errechenbar ist. Es sind zudem Eingaben, wie beispielsweise tatsächlich konsumierte Speisen und Getränke sowie tatsächlich durchgeführte Aktivitäten (Sport und/oder Alltagsbewegung), zu einer Generierung einer Rezeptur und/oder Dosierung berücksichtigbar. Es kann somit vorteilhaft eine exakte Justierung der Rezeptur und/oder Dosierung ermöglicht werden.

Des Weiteren wird vorgeschlagen, dass die Vorrichtung zumindest eine Vorratseinheit zu einer Bevorratung von zumindest einem Bestandteil zur Herstellung der individuell angepassten Verabreichungs- und/oder Betriebshilfsstoffeinheit umfasst. Die Vorratseinheit umfasst hierzu vorzugsweise zumindest einen Vorratsbehälter, in dem der zumindest eine Bestandteil zur Herstellung der individuell angepassten Verabreichungs- und/oder Betriebshilfsstoffeinheit anordenbar ist. Der Vorratsbehälter kann hierbei als Fluidvorratsbehälter und/oder als Feststoffvorratsbehälter ausgebildet sein. Besonders bevorzugt ist die Vorratseinheit wiederbefüllbar ausgebildet. Es ist jedoch auch denkbar, dass die Vorratseinheit als Einmalvorratseinheit ausgebildet ist, die nach einem Gebrauch gegen eine weitere Vorratseinheit ausgetauscht wird. Hierbei ist es denkbar, dass die Vorratseinheit als Vorratsaustauschpatroneneinheit ausgebildet ist. Zudem ist es denkbar, dass die Vorrichtung alternativ oder zusätzlich zur Vorratseinheit eine Direktverarbeitungseinheit umfasst, die dazu vorgesehen ist, Rohstoffe zu einer Herstellung der Verabreichungs- und/oder Betriebshilfsstoffeinheit direkt zu verarbeiten, insbesondere zu fräsen, zu schleifen, zu schmelzen, zu drehen, zu spritzen o. dgl., und diese beispielsweise einem Herstellungsprozess der Verabreichungs- und/oder Betriebshilfsstoffeinheit mittels einer Fördereinheit zukommen zu lassen. Mittels der erfindungsgemäßen Ausgestaltung kann konstruktiv einfach ein Bestandteil zur Herstellung der individuell angepassten Verabreichungs- und/oder Betriebshilfsstoffeinheit bevorratet werden, so dass eine Herstellung der Verabreichungs- und/oder Betriebshilfsstoffeinheit zumindest im Wesentlichen ortsunabhängig erfolgen kann. Somit kann eine hohe Flexibilität der Vorrichtung erreicht werden.

Ferner wird vorgeschlagen, dass die Vorratseinheit zumindest zwei getrennte Kammern zu einer Bevorratung von zumindest zwei unterschiedlichen Bestandteilen der individuell angepassten Verabreichungs- und/oder Betriebshilfsstoffeinheit aufweist. Besonders bevorzugt umfasst die Vorratseinheit eine von zwei abweichende Vielzahl an Kammern zu einer Bevorratung von unterschiedlichen Bestandteilen der individuell angepassten Verabreichungs- und/oder Betriebshilfsstoffeinheit. Die zwei getrennten Kammern sind hierbei in einem gemeinsamen Vorratsbehälter der Vorratseinheit anordenbar. Es ist jedoch auch denkbar, dass die Vorratseinheit zu einer Bildung der zwei getrennten Kammern zumindest zwei getrennt ausgebildete Vorratsbehälter aufweist. Die Vorratsbehälter sind insbesondere austauschbar an einer Gehäuseeinheit und/oder an einer Aufnahmeeinheit der Vorrichtung anordenbar. Somit kann vorteilhaft eine große Vielfalt an möglichen Zusammensetzungen der Verabreichungs- und/oder Betriebshilfsstoffeinheit ermöglicht werden. Zudem kann vorteilhaft ein unkomplizierter Austausch von verschiedenen Vorratsbehältern mit unterschiedlichen Bestandteilen der individuell angepassten Verabreichungs- und/oder Betriebshilfsstoffeinheit ermöglicht werden.

Zudem wird vorgeschlagen, dass die Vorrichtung zumindest eine Dosiereinheit zu einer Dosierung von zumindest einem Bestandteil der individuell angepassten Verabreichungs- und/oder Betriebshilfsstoffeinheit und/oder zu einer Ausgabedosierung der individuell angepassten Verabreichungs- und/oder Betriebshilfsstoffeinheit umfasst. Besonders bevorzugt umfasst die Dosiereinheit zumindest eine Dosierdüse. Die Dosierdüse ist vorzugsweise dazu vorgesehen, in Abhängigkeit von einer mittels der Rezeptur- und/oder Dosierungsgenerierungseinheit generierten Rezeptur eine Menge des Bestandteils der der individuell angepassten Verabreichungs- und/oder Betriebshilfsstoffeinheit abzugeben. Es ist jedoch auch denkbar, dass die Dosierdüse alternativ oder zusätzlich dazu vorgesehen ist, eine Menge der individuell angepassten Verabreichungs- und/oder Betriebshilfsstoffeinheit abzugeben. Besonders bevorzugt umfasst die Dosiereinheit ein Ausgabedosierelement, wie beispielsweise eine Ausgabedosierdüse, das dazu vorgesehen ist, in Abhängigkeit von der zumindest einen individuellen Kenngröße eine Menge der individuell angepassten Verabreichungs- und/oder Betriebshilfsstoffeinheit abzugeben. Mittels der erfindungsgemäßen Ausgestaltung der Vorrichtung kann vorteilhaft eine präzise Dosierung von zumindest einem Bestandteil der individuell angepassten Verabreichungs- und/oder Betriebshilfsstoffeinheit und/oder eine präzise Ausgabedosierung der individuell angepassten Verabreichungs- und/oder Betriebshilfsstoffeinheit erfolgen, die besonders vorteilhaft an ein individuelles Bedürfnis und/oder an eine individuelle Anwendung angepasst ist.

Des Weiteren wird vorgeschlagen, dass die Vorrichtung zumindest eine Mischeinheit zu einer Mischung von Bestandteilen der individuell angepassten Verabreichungs- und/oder Betriebshilfsstoffeinheit aufweist. Vorzugsweise ist die Mischeinheit dazu vorgesehen, aus Ausgangsstoffen, insbesondere aus Bestandteilen der Verabreichungs- und/oder Betriebshilfsstoffeinheit, ein Gemenge herzustellen, das die Verabreichungs- und/oder Betriebshilfsstoffeinheit bildet. Das mittels der Mischeinheit erzeugbare Gemenge kann hierbei gasförmig, flüssig oder fest ausgebildet sein. Die Mischeinheit ist als Zwangsmischeinheit, als Freifallmischeinheit, als Rührmischeinheit, als Knetmischeinheit, als Strömungsmischeinheit, als Mischpumpenmischeinheit oder als eine andere, einem Fachmann als sinnvoll erscheinende Mischeinheit ausbildbar. Die Dosiereinheit steuert und/oder regelt vorzugsweise in Abhängigkeit von einer mittels der Rezeptur- und/oder Dosierungsgenerierungseinheit generierten Rezeptur und/oder Dosierung welche Menge eines bestimmten Bestandteils der individuell angepassten Verabreichungs- und/oder Betriebshilfsstoffeinheit in die Mischeinheit gelangt. Mittels der erfindungsgemäßen Ausgestaltung der Vorrichtung kann eine vorteilhafte Vermengung von Bestandteilen der individuell angepassten Verabreichungs- und/oder Betriebshilfsstoffeinheit ermöglicht werden.

Ferner wird vorgeschlagen, dass die Vorrichtung zumindest eine Eingabeeinheit zu einer manuellen Eingabe von zumindest einer individuell abgestimmten Rezeptur und/oder Dosierung zur Herstellung der individuell angepassten Verabreichungs- und/oder Betriebshilfsstoffeinheit umfasst. Die Eingabeeinheit ist hierbei als akustische, als haptische und/oder als optische Eingabeeinheit ausbildbar. Bei einer Ausgestaltung der Eingabeeinheit als reine akustische Eingabeeinheit ist die individuell abgestimmte Rezeptur und/oder Dosierung mittels eines akustischen Signals, wie beispielsweise mittels einer Sprachsteuerung o. dgl., eingebbar. Bei einer Ausgestaltung der Eingabeeinheit als reine haptische Eingabeeinheit ist die individuell abgestimmte Rezeptur und/oder Dosierung mittels eines haptischen Signals, wie beispielsweise mittels einer Tastatur, mittels eines Wählschalters, mittels eines berührungsempfindlichen Displays o. dgl., eingebbar. Bei einer Ausgestaltung der Eingabeeinheit als reine optische Eingabeeinheit ist die individuell abgestimmte Rezeptur und/oder Dosierung mittels eines optischen Signals, wie beispielsweise mittels eines Barcodes, mittels einer Kamera o. dgl., eingebbar. Es ist jedoch auch denkbar, dass die Eingabeeinheit als eine Kombination aus einer akustischen, haptischen und/oder optischen Eingabeeinheit ausgebildet ist. Zudem ist es denkbar, dass die Rezeptur mittels eines anderen, einem Fachmann als sinnvoll erscheinenden Verfahrens zur Datenübertragung eingebbar ist, wie beispielsweise mittels eines RFID-Verfahrens, mittels eines NFC-Verfahrens, mittels USB, mittels FireWire o. dgl. Mittels der erfindungsgemäßen Ausgestaltung der Vorrichtung kann vorteilhaft eine komfortable Bedienung der Vorrichtung ermöglicht werden. Zudem kann vorteilhaft beispielsweise ein Abgleich von einer freigegebenen Rezeptur mit einer mittels der Rezeptur- und/oder Dosierungsgenerierungseinheit erzeugten Rezeptur und/oder Dosierung erfolgen. Somit kann vorteilhaft eine hohe Sicherheit gegen eine Überdosierung oder eine falsche Rezeptur erreicht werden.

Des Weiteren wird vorgeschlagen, dass die Vorrichtung zumindest eine Auswerteeinheit umfasst, die dazu vorgesehen ist, die zumindest eine individuell eingegebene Kenngröße zumindest hinsichtlich einer Generierung einer individuell abgestimmten Rezeptur und/oder Dosierung auszuwerten. Besonders bevorzugt ist die Auswerteeinheit dazu vorgesehen, Ist-Daten mit Soll-Daten zu vergleichen und anhand der zumindest einen individuell eingegebenen Kenngröße eine individuell abgestimmte Rezeptur und/oder Dosierung zu ermöglichen. Somit ist vorzugsweise eine Überwachung einer Wirkung der Verabreichungs- und/oder Betriebshilfsstoffeinheit möglich.

Ferner wird vorgeschlagen, dass die Vorrichtung zumindest eine Sensoreinheit aufweist, die dazu vorgesehen ist, die zumindest eine individuelle Kenngröße zu ermitteln, die zu einer Generierung einer individuell abgestimmten Rezeptur und/oder Dosierung nutzbar ist. Besonders bevorzugt umfasst die Sensoreinheit zumindest ein Sensorelement zu einer Erfassung der zumindest einen individuellen Kenngröße. Das Sensorelement ist hierbei insbesondere dazu vorgesehen, zumindest eine Vitalkenngröße eines Individuums, eine Gesundheitszustandskenngröße eines Individuums, eine Leistungskenngröße eines Individuums, eine Anwendungskenngröße o. dgl. zu erfassen. Somit ist das Sensorelement beispielsweise als Pulserfassungselement, als Blutdruckerfassungselement, als Herzrhythmuserfassungselement, als Blutbilderfassungselement, als Zahnzustandserfassungselement, als Hautverträglichkeitserfassungselement, als Schritterfassungselement, als Schlafverhaltenserfassungselement, als Biorhythmuserfassungselement, als Blutzuckerspiegelerfassungselement, als Insulinspiegelerfassungselement, als Nährstofferfassungselement, als Stoffwechselerfassungselement, als Feuchtigkeitserfassungselement, als Blutgerinnungserfassungselement, als Schmierstoffviskositätserfassungselement, als Bauteilleimartverbindungserfassungselement o. dgl. ausgebildet. Mittels der erfindungsgemäßen Ausgestaltung kann vorteilhaft eine zumindest teilweise automatische Erfassung einer individuellen Kenngröße erfolgen. Somit kann ein besonders hoher Bedienkomfort erreicht werden, da eine Generierung einer Rezeptur und/oder einer Dosierung zumindest weitestgehend unabhängig von einer manuellen Eingabe einer individuellen Kenngröße ist.

Zudem wird vorgeschlagen, dass die Vorrichtung zumindest eine Kommunikationseinheit aufweist, die dazu vorgesehen ist, mit zumindest einer externen Einheit elektronische Daten auszutauschen. Die Kommunikationseinheit ist vorzugsweise als kabellose Kommunikationseinheit ausgebildet. Hierbei kann die Kommunikationseinheit als WLAN-Kommunikationseinheit, als Bluetooth-Kommunikationseinheit, als Funk-Kommunikationseinheit, als RFID-Kommunikationseinheit, als NFC-Einheit, als Infrarot-Kommunikationseinheit, als Mobilfunknetz-Kommunikationseinheit o. dgl. ausgebildet sein. Besonders bevorzugt ist die Kommunikationseinheit zu einer bidirektionalen Datenübertragung vorgesehen. In einer alternativen Ausgestaltung ist die Kommunikationseinheit als kabelgebundene Kommunikationseinheit ausgebildet, wie beispielsweise als LAN-Kommunikationseinheit, als USB-Kommunikationseinheit o. dgl. Somit ist vorteilhaft ein Austausch von elektronischen Daten mit einer externen Einheit, wie beispielsweise mit einem Smartphone, möglich. Die externe Einheit weist bevorzugt eine App zu einer Kommunikation mit der Kommunikationseinheit auf, die dazu vorgesehen ist, eine Eingabe und/oder eine automatische Erfassung von der zumindest einen individuellen Kenngröße zu ermöglichen. Hierbei kann die App beispielsweise auch einen individuellen Trainingsplan berücksichtigen. Es ist jedoch auch denkbar, dass die Vorrichtung mittels der Kommunikationseinheit mit einer verschieden von einem Smartphone ausgebildeten Einheit elektronische Daten austauschen kann, wie beispielsweise mit einem PC, mit einem Laptop, mit einem Server o. dgl. Somit kann vorteilhaft eine Übermittlung und/oder ein Austausch von elektronischen Daten ermöglicht werden, um eine generierte Rezeptur und/oder Dosierung zu überprüfen, zu ändern und/oder zu validieren, insbesondere durch eine Fachperson, wie beispielsweise einen Arzt, einen Apotheker, einen Produktproduzent, einen Sportcoach o. dgl. Somit kann vorteilhaft eine sichere Rezeptur und/oder Dosierung generiert werden. Zudem kann vorteilhaft eine Überwachung der generierten Rezeptur und/oder Dosierung ermöglicht werden. Zudem ist vorteilhaft eine Überwachung einer Person möglich, die eine erfindungsgemäße Vorrichtung am Körper trägt. Zudem kann vorteilhaft infolge einer Erfassung von individuellen Kenngrößen einer Person, die eine erfindungsgemäße Vorrichtung am Körper trägt, eine Vitalfunktionsüberwachung erfolgen, wobei bei einer Erkennung einer Notfallsituation über die Kommunikationseinheit ein Notrufsignal mit auswertbaren Daten an eine Notfallstation versandt wird. Zudem kann vorteilhaft beispielsweise infolge einer Kommunikation mit einem Smartphone, das eine Rezeptur- und/oder Dosierungsapp aufweist, eine Rezeptur und/oder eine Dosierung einer individuell angepassten Verabreichungseinheit mittels der Rezeptur- und/oder Dosierungsapp generiert werden, die an die Vorrichtung übermittelbar ist und/oder anhand derer manuell eine individuell angepasste Verabreichungseinheit herstellbar ist.

Des Weiteren wird vorgeschlagen, dass die Vorrichtung zumindest eine Umgebungskenngrößenerfassungseinheit umfasst, die dazu vorgesehen ist, zumindest eine Umgebungskenngröße zu einer Generierung einer individuell abgestimmten Rezeptur und/oder Dosierung zu erfassen. Die Umgebungskenngrößenerfassungseinheit umfasst zumindest ein Umgebungskenngrößensensorelement, das dazu vorgesehen ist, eine Umgebungskenngröße zu erfassen. Das Umgebungskenngrößensensorelement ist als Wettersensorelement, als Pollenbelastungssensorelement, als Umgebungstemperatursensorelement, als Umgebungsluftdrucksensorelement, als Sonnenscheindauersensorelement, als Niederschlagsensorelement, als UV.-Sensorelement o. dgl. ausbildbar. Es ist zudem auch denkbar, dass die Rezeptur- und/oder Dosierungsgenerierungseinheit der Vorrichtung alternativ oder zusätzlich eine Datenbank umfasst, in der Umgebungskenngrößen hinterlegbar sind, die bei einer Generierung einer Rezeptur und/oder einer Dosierung berücksichtigbar sind. Mittels der erfindungsgemäßen Ausgestaltung der Vorrichtung können vorteilhaft Umweltfaktoren bei einer Generierung einer Rezeptur und/oder einer Dosierung berücksichtigt werden. Somit ist beispielsweise vorteilhaft eine Rezeptur und/oder Dosierung an eine Pollenflugvorhersage und/oder eine konkrete Pollenbelastung anpassbar.

Ferner wird vorgeschlagen, dass die Vorrichtung zumindest eine Rezepturausgabeeinheit aufweist, die dazu vorgesehen ist, zumindest eine individuelle abgestimmte Rezeptur und/oder Dosierung auszugeben. Die Rezepturausgabeeinheit ist hierbei als optische und/oder als akustische Rezepturausgabeeinheit ausgebildet. Bevorzugt ist die Rezepturausgabeeinheit als Rezepturdruckereinheit ausgebildet. Es ist jedoch auch denkbar, dass die Rezepturausgabeeinheit alternativ oder zusätzlich als Displayeinheit und/oder als Lautsprechereinheit ausgebildet ist. Weitere, einem Fachmann als sinnvoll erscheinende Ausgestaltungen der Rezepturausgabeeinheit sind ebenfalls denkbar. Mittels der erfindungsgemäßen Ausgestaltung kann vorteilhaft eine Ausgabe einer Rezeptur und/oder einer Dosierung erfolgen, um eine manuelle Vermengung von Bestandteilen der Verabreichungs- und/oder Betriebshilfsstoffeinheit zu ermöglichen.

Zudem wird vorgeschlagen, dass die Vorrichtung zumindest eine Verpackungserzeugungseinheit umfasst, die dazu vorgesehen ist, eine Verpackung der individuell angepassten Verabreichungs- und/oder Betriebshilfsstoffeinheit herzustellen. Die Verpackungserzeugungseinheit kann zusätzlich dazu vorgesehen sein, eine Umverpackung der individuell angepassten Verabreichungs- und/oder Betriebshilfsstoffeinheit herzustellen. Bevorzugt ist die Verpackungserzeugungseinheit dazu vorgesehen, die individuell angepasste Verabreichungs- und/oder Betriebshilfsstoffeinheit nach einer Herstellung zu verpacken und/oder umzuverpacken. Mittels der erfindungsgemäßen Ausgestaltung der Vorrichtung kann vorteilhaft ein sicherer Schutz der individuell angepassten Verabreichungs- und/oder Betriebshilfsstoffeinheit nach einer Herstellung erreicht werden. Somit ist die individuell angepasste Verabreichungs- und/oder Betriebshilfsstoffeinheit komfortabel lager- und/oder transportierbar.

Des Weiteren wird vorgeschlagen, dass die Vorrichtung zumindest eine Injektionseinheit zu einer Injektion zumindest einer individuell angepassten Verabreichungseinheit aufweist. Die Injektionseinheit umfasst vorzugsweise zumindest ein Injektionselement, insbesondere eine Injektionsnadel, zu einer Injektion zumindest einer individuell angepassten Verabreichungseinheit. Hierbei kann die Vorrichtung derart ausgebildet sein, dass eine entsprechende Dosierung automatisch übermittelbar ist oder manuell eingebbar ist. Somit ist beispielsweise eine Verabreichung von Injektionen, wie beispielsweise bei einem Diabetiker, oder bei Impfungen eine individuelle Injektion vornehmbar. Hierbei ist die Vorrichtung an einer Haut eines Individuums anordenbar, um beispielsweise die Verabreichungseinheit subkutan oder direkt ins Blut abzugeben. Mittels der erfindungsgemäßen Ausgestaltung kann vorteilhaft eine direkte Verabreichung einer individuell angepassten Verabreichungseinheit erreicht werden.

Zudem wird eine Rezeptur- und/oder Dosierungsgenerierungseinheit einer erfindungsgemäßen Vorrichtung vorgeschlagen. Die Rezeptur- und/oder Dosierungsgenerierungseinheit ist vorzugsweise als autarke Einheit ausgebildet, die mittels der Kommunikationseinheit mit der Vorrichtung elektronische Daten austauschen kann. Somit ist die Rezeptur- und/oder Dosierungsgenerierungseinheit besonders bevorzugt lösbar an einer Halteeinheit der Vorrichtung anordenbar. Somit kann vorteilhaft eine hohe Flexibilität der Rezeptur- und/oder Dosierungsgenerierungseinheit erreicht werden.

Ferner wird ein Verfahren zu einem Betrieb der erfindungsgemäßen Vorrichtung vorgeschlagen, wobei eine individuelle Generierung einer Rezeptur und/oder Dosierung zu einer Herstellung von zumindest einer individuell angepassten Verabreichungs- und/oder Betriebshilfsstoffeinheit und/oder zu einer Verabreichung von zumindest einer individuell angepassten Verabreichungseinheit erfolgt. Mittels des erfindungsgemäßen Verfahrens kann vorteilhaft eine Rezeptur und/oder Dosierung generiert werden, die an spezielle Bedürfnisse angepasst ist. Somit kann vorteilhaft eine Unter- oder

Überdosierung der Verabreichungs- und/oder Betriebshilfsstoffeinheit vermieden werden. Somit kann ein vorteilhaftes Nutzprofil der Verabreichungs- und/oder Betriebshilfsstoffeinheit ermöglicht werden.

Zudem wird vorgeschlagen, dass die Generierung einer Rezeptur und/oder Dosierung mittels einer manuellen Eingabe von der zumindest einen individuellen Kenngröße durchführbar ist. Bevorzugt ist die zumindest eine individuelle Kenngröße mittels der Eingabeeinheit der Vorrichtung eingebbar. Anhand der manuellen Eingabe ist mittels der Vorrichtung eine Rezeptur und/oder Dosierung generierbar, anhand dessen automatisch mittels der Vorrichtung eine Verabreichungs- und/oder Betriebshilfsstoffeinheit herstellbar ist oder anhand dessen einem Bediener eine Anleitung zur Verfügung stellbar ist, die es dem Bediener ermöglicht Verabreichungs- und/oder Betriebshilfsstoffeinheit infolge einer Vermengung, insbesondere einer manuellen Vermengung, herzustellen. Mittels des erfindungsgemäßen Verfahrens ist vorteilhaft eine einfache Eingabe von individuellen Kenngrößen möglich.

Des Weiteren wird vorgeschlagen, dass eine Erfassung von der zumindest einen individuellen Kenngröße zu einer Generierung einer Rezeptur und/oder Dosierung zumindest teilweise automatisch erfolgt. Durch zumindest ein geeignetes Sensorelement sind individuelle Kenngrößen und/oder weitere Werte ermittelbar und/oder analysierbar, die zu einer Generierung einer individuell angepassten Rezeptur und/oder Dosierung nutzbar sind. Somit kann vorteilhaft ein hoher Bedienkomfort erreicht werden.

Ferner wird vorgeschlagen, dass die Herstellung von individuell angepassten Verabreichungs- und/oder Betriebshilfsstoffeinheiten und/oder die Verabreichung von individuell angepassten Verabreichungseinheiten zumindest in Abhängigkeit von der zumindest einen individuellen Kenngröße zumindest teilweise automatisch erfolgen/erfolgt. Hierbei wird vorzugsweise infolge einer manuellen Eingabe und/oder einer automatischen Erfassung der zumindest einen individuellen Kenngröße ein Rezeptur- und/oder Dosierungsprofil erstellt und analysiert. Vorzugsweise wird auf dieser Grundlage eine individuelle Rezeptur und/oder Dosierung generiert. Infolge von wiederkehrenden Eingaben von individuellen Kenngrößen sind/ist das Rezeptur- und/oder Dosierungsprofil an sich ändernde Gegebenheiten automatisch anpassbar. Die Eingaben können auch durch einen Dritten z.B. Arzt/Fachperson erfolgen, validiert und/oder angepasst werden. Somit kann vorteilhaft eine exakte Justierung der Rezeptur und/oder Dosierung erreicht werden.

Zudem wird vorgeschlagen, dass in zumindest einem Verfahrensschritt ein Abgleich und/oder eine Überprüfung der individuell abgestimmten Rezeptur und/oder Dosierung erfolgt. Zusätzliche ist es denkbar, dass äußeren Faktoren oder Daten wie beispielsweise Wetter, Ort, Temperatur, Pollenflug, Bewegungsverhalten, Ernährungsverhalten, berücksichtigt werden und in die Generierung der Rezeptur und/oder Dosierung einfließen. Mittels des erfindungsgemäßen Verfahrens kann somit zuverlässig eine sichere Generierung einer Rezeptur und/oder Dosierung ermöglicht werden.

Des Weiteren wird vorgeschlagen, dass der Abgleich und/oder die Überprüfung der individuell abgestimmten Rezeptur und/oder Dosierung infolge einer elektronischen Datenübertragung erfolgt. Hierbei sind/ist die individuell abgestimmte Rezeptur und/oder Dosierung insbesondere mittels der Kommunikationseinheit der Vorrichtung an Dritte, wie beispielsweise einen Arzt und/oder Apotheker, übermittelbar. Vorzugsweise erfolgt nach einer Überprüfung und/oder Validierung der übermittelten Rezeptur und/oder Dosierung durch Dritte oder eine Software eine Rückübermittelung und die individuell angepasste Verabreichungs- und/oder Betriebshilfsstoffeinheit ist mittels der Vorrichtung entsprechend verabreichbar und/oder herstellbar. Somit kann vorteilhaft sichergestellt werden, dass eine unschädliche Rezeptur und/oder Dosierung generiert wird.

Die erfindungsgemäße Vorrichtung, die erfindungsgemäße Rezeptur- und/oder Dosierungsgenerierungseinheit und/oder das erfindungsgemäße Verfahren sollen/soll hierbei nicht auf die oben beschriebene Anwendung und Ausführungsform beschränkt sein. Insbesondere können/kann die erfindungsgemäße Vorrichtung, die erfindungsgemäße Rezeptur- und/oder Dosierungsgenerierungseinheit und/oder das erfindungsgemäße Verfahren zu einer Erfüllung einer hierin beschriebenen Funktionsweise eine von einer hierin genannten Anzahl von einzelnen Elementen, Bauteilen und Einheiten abweichende Anzahl aufweisen.

### Zeichnungen

Weitere Vorteile ergeben sich aus der folgenden Zeichnungsbeschreibung. In den Zeichnungen sind acht Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine Detailansicht einer erfindungsgemäßen Vorrichtung in einer schematischen Darstellung,
- Fig. 2: eine Detailansicht einer alternativen erfindungsgemäßen Vorrichtung in Form einer Applikatorvorrichtung in einer schematischen Darstellung,
- Fig. 3: eine Detailansicht einer weiteren alternativen erfindungsgemäßen Vorrichtung in Form einer dosenartigen Vorrichtung in einer schematischen Darstellung,
- Fig. 4: eine Detailansicht einer weiteren alternativen erfindungsgemäßen Vorrichtung in Form einer kapselartigen Vorrichtung in einer schematischen Darstellung,
- Fig. 5: eine Detailansicht einer weiteren alternativen erfindungsgemäßen Vorrichtung in Form einer tubenartigen Vorrichtung in einer schematischen Darstellung,
- Fig. 6: eine Detailansicht einer weiteren alternativen erfindungsgemäßen Vorrichtung in Form einer flaschenartigen Vorrichtung in einer schematischen Darstellung und
- Fig. 7: eine Detailansicht einer weiteren alternativen erfindungsgemäßen Vorrichtung in Form einer Automatenvorrichtung in einer schematischen Darstellung.

### Beschreibung der Ausführungsbeispiele

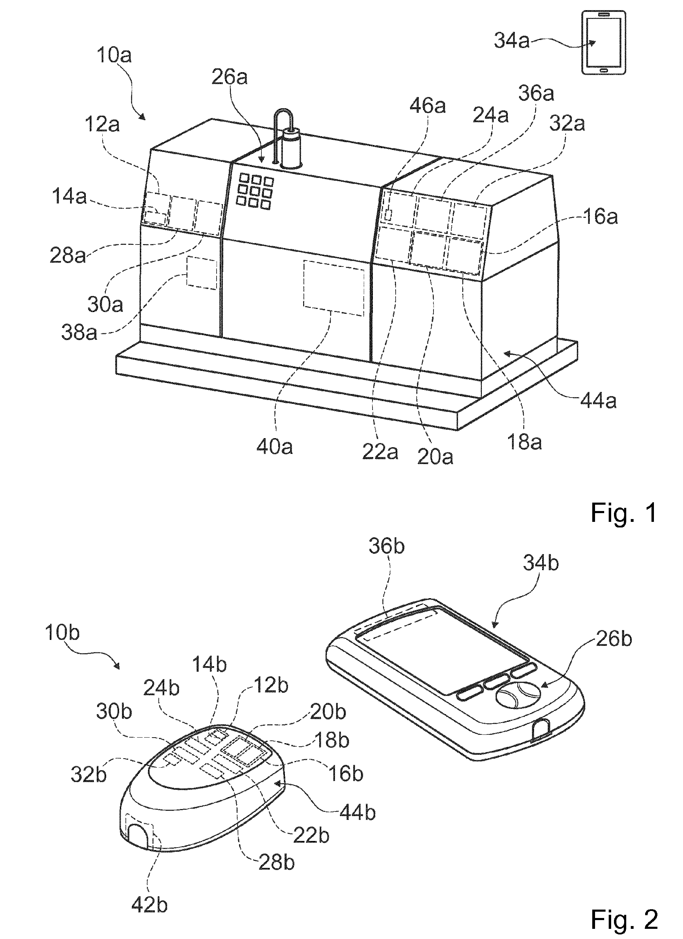

Figur 1 zeigt eine Vorrichtung 10a zu einer Herstellung von zumindest einer individuell angepassten Verabreichungs- und/oder Betriebshilfsstoffeinheit (hier nicht näher dargestellt) und/oder zu einer Verabreichung von zumindest einer individuell angepassten Verabreichungseinheit (hier nicht näher dargestellt) in Abhängigkeit von zumindest einer individuellen Kenngröße. Die Vorrichtung 10a ist dazu vorgesehen, Verabreichungs- und/oder Betriebshilfsstoffeinheit herzustellen, die als Pulver, als Tablette, als Brei, als Flüssigkeit, als Creme o. dgl. ausgebildet ist. Zudem ist die Vorrichtung 10a alternativ oder zusätzlich dazu vorgesehen, einem Individuum, von dem die zumindest eine individuelle Kenngröße abfragbar oder erfassbar ist, zumindest die mittels der Vorrichtung 10a hergestellte Verabreichungseinheit zu verabreichen. Die Vorrichtung 10a ist in dem in Figur 1 dargestellten Ausführungsbeispiel als 3D-Druckervorrichtung ausgebildet. Somit ist die Verabreichungs- und/oder Betriebshilfsstoffeinheit mittels der Vorrichtung 10a konsumfertig herstellbar. Es ist jedoch auch denkbar, dass die Vorrichtung 10a eine andere, einem Fachmann als sinnvoll erscheinende Ausgestaltung aufweist.

Des Weiteren weist die Vorrichtung 10a zumindest eine Rezeptur- und/oder Dosierungsgenerierungseinheit 12a auf, die dazu vorgesehen ist, eine Rezeptur und/oder eine Dosierung der Verabreichungs- und/oder Betriebshilfsstoffeinheit in Abhängigkeit von der zumindest einen individuellen Kenngröße zu generieren. Hierbei ist die Rezeptur- und/oder Dosierungsgenerierungseinheit 12a dazu vorgesehen, infolge eines von einem Zustand eines Individuums abhängigen Bedarfs eine individuell abgestimmte Rezeptur und/oder Dosierung der Verabreichungs- und/oder Betriebshilfsstoffeinheit in Abhängigkeit von der zumindest einen individuellen Kenngröße zu generieren, insbesondere eine individuell abgestimmte Rezeptur und/oder Dosierung von Medikamenten, Nahrungsmitteln Nahrungsergänzungsmitteln ,Cremes, Kosmetika o. dgl. Die Rezeptur- und/oder Dosierungsgenerierungseinheit 12a ist hierbei an einer Gehäuseeinheit 44a der Vorrichtung 10a angeordnet. Hierbei umschließt die Gehäuseeinheit 44a die Rezeptur- und/oder Dosierungsgenerierungseinheit 12a. Somit ist die Rezeptur- und/oder Dosierungsgenerierungseinheit 12a in der Gehäuseeinheit 44a angeordnet. Es ist jedoch auch denkbar, dass die Rezeptur- und/oder Dosierungsgenerierungseinheit 12a lösbar an der Gehäuseeinheit 44a anordenbar ist und mittels einer Kommunikationseinheit 32a der Vorrichtung 10a elektronische Daten zu einer Generierung von einer Rezeptur und/oder eine Dosierung der Verabreichungs- und/oder Betriebshilfsstoffeinheit in Abhängigkeit von der zumindest einen individuellen Kenngröße mit der Vorrichtung 10a austauscht und/oder an die Vorrichtung 10a überträgt. Die Rezeptur- und/oder Dosierungsgenerierungseinheit 12a umfasst zumindest eine Steuer- und/oder Regeleinheit 14a, die zumindest ein Betriebsprogramm aufweist, das in Abhängigkeit einer Eingabe von der zumindest einen individuellen Kenngröße ein Rezeptur- und/oder Dosierungsprofil erstellt. Hierbei ist mittels des Betriebsprogramms der Steuer- und/oder Regeleinheit 14a infolge einer Eingabe, insbesondere einer manuellen und/oder automatischen Eingabe, von der zumindest einen individuellen Kenngröße ein Rezeptur- und/oder Dosierungsprofil erstellbar und/oder analysierbar. Auf dieser Grundlage ist mittels der Steuer- und/oder Regeleinheit 14a eine individuelle Rezeptur und/oder Dosierung ermittelbar und/oder ausgebbar. Durch regelmäßige Eingaben von individuellen Kenngrößen ist das Rezeptur- und/oder Dosierungsprofil an neue Gegebenheiten anpassbar. Somit ist die Rezeptur und/oder Dosierung der Verabreichungs- und/oder Betriebshilfsstoffeinheit exakt justierbar.

Ferner weist die Vorrichtung 10a zumindest eine Eingabeeinheit 26a zu einer manuellen Eingabe von zumindest einer individuell abgestimmten Rezeptur und/oder Dosierung zur Herstellung der individuell angepassten Verabreichungs- und/oder Betriebshilfsstoffeinheit auf. Die Eingabeeinheit 26a ist somit als Manuelleingabeeinheit ausgebildet. Hierbei umfasst die Eingabeeinheit 26a zumindest ein berührungsempfindliches Display zu einer manuellen Eingabe einer individuell abgestimmten Rezeptur und/oder Dosierung anhand der die Verabreichungs- und/oder Betriebshilfsstoffeinheit mittels der Vorrichtung 10a herstellbar und/oder verabreichbar ist. Es ist jedoch auch denkbar, dass die Eingabeeinheit 26a alternativ oder zusätzlich zumindest ein Bedienelement, wie beispielsweise ein Taster, ein Schalter, ein Wahlschalter, eine Tastatur o. dgl., aufweist, mittels dessen eine manuelle Eingabe der zumindest einen individuellen Kenngröße erfolgen kann.

Zudem weist die Vorrichtung 10a zumindest eine Auswerteeinheit 28a auf, die dazu vorgesehen ist, die zumindest eine individuell eingegebene Kenngröße zumindest hinsichtlich einer Generierung einer individuell abgestimmten Rezeptur und/oder Dosierung auszuwerten. Die Auswerteeinheit 28a ist hierbei dazu vorgesehen, Ist-Daten mit Soll-Daten zu vergleichen und anhand der zumindest einen individuell eingegebenen Kenngröße eine individuell abgestimmte Rezeptur und/oder Dosierung zu ermöglichen.

Die Vorrichtung 10a umfasst ferner zumindest eine Sensoreinheit 30a, die dazu vorgesehen ist, die zumindest eine individuelle Kenngröße zu ermitteln, die zu einer Generierung einer individuell abgestimmten Rezeptur und/oder Dosierung nutzbar ist. Hierzu umfasst die Sensoreinheit 30a zumindest ein Sensorelement (hier nicht näher dargestellt), das zu einer Ermittlung der individuellen Kenngröße vorgesehen ist. Das Sensorelement kann hierbei als berührungsloses oder als kontaktbedingtes Sensorelement ausgebildet sein. Zudem ist mittels eines Zusammenwirkens der Sensoreinheit 30a und der Auswerteeinheit 28a eine Dosierung und/oder eine Verabreichung der Verabreichungs- und/oder Betriebshilfsstoffeinheit möglich.

Des Weiteren umfasst die Vorrichtung 10a zumindest eine Kommunikationseinheit 32a, die dazu vorgesehen ist, mit zumindest einer externen Einheit 34a elektronische Daten auszutauschen. Die Kommunikationseinheit 32a ist als drahtlose Kommunikationseinheit ausgebildet. Es ist jedoch auch denkbar, dass die Kommunikationseinheit 32a alternativ oder zusätzlich als kabelgebundene Kommunikationseinheit ausgebildet ist. Die externe Einheit 34a kann beispielsweise als Smartphone ausgebildet sein, auf dem eine Software-Applikation installiert ist, mittels derer eine Eingabe und/oder eine Erfassung der zumindest einen individuellen Kenngröße erfolgen kann, die mittels der Kommunikationseinheit 32a an die Rezeptur- und/oder Dosierungsgenerierungseinheit 12a übertragbar ist. Somit ist eine Herstellung einer Verabreichungs- und/oder Betriebshilfsstoffeinheit und/oder eine Verabreichung einer mittels der Vorrichtung 10a hergestellten Verabreichungseinheit mittels der externen Einheit 34a steuer- und/oder regelbar. Zudem ist es denkbar, dass mittels einer Kommunikation der Vorrichtung 10a und der externen Einheit 34a eine vollständige Steuerung- und/oder Regelung der Vorrichtung 10a erfolgen kann.

Die Vorrichtung 10a weist zudem zumindest eine

Umgebungskenngrößenerfassungseinheit 36a auf, die dazu vorgesehen ist, zumindest eine Umgebungskenngröße zu einer Generierung einer individuell abgestimmten Rezeptur und/oder Dosierung zu erfassen. Die Umgebungskenngrößenerfassungseinheit 36a umfasst hierzu zumindest ein Umgebungskenngrößensensorelement (hier nicht näher dargestellt), das zu einer Erfassung zumindest einer Umgebungskenngröße vorgesehen ist, die bei einer Generierung einer Rezeptur und/oder Dosierung berücksichtigbar ist. Zudem ist mittels eines Zusammenwirkens der Umgebungskenngrößenerfassungseinheit 36a und der Auswerteeinheit 28a eine Dosierung und/oder eine Verabreichung der Verabreichungs- und/oder Betriebshilfsstoffeinheit möglich. Beispielsweise sind/ist mittels des Umgebungskenngrößensensorelements eine Pollenbelastung und/oder Allergene erfassbar, die bei einer Generierung einer Rezeptur und/oder Dosierung mittels der Rezeptur- und/oder Dosierungsgenerierungseinheit 12a und/oder bei einer Verabreichung einer Verabreichungseinheit berücksichtigbar sind/ist.

Ferner umfasst die Vorrichtung 10a zumindest eine Rezepturausgabeeinheit 38a, die dazu vorgesehen ist, zumindest eine mittels der Rezeptur- und/oder Dosierungsgenerierungseinheit 12a generierte, individuelle abgestimmte Rezeptur und/oder Dosierung auszugeben. Die Rezepturausgabeeinheit 38a ist hierbei dazu vorgesehen, die individuelle abgestimmte Rezeptur und/oder Dosierung als Ausdruck abzugeben. Es ist jedoch auch denkbar, dass die Rezepturausgabeeinheit 38a dazu vorgesehen ist, die individuelle abgestimmte Rezeptur und/oder Dosierung auf eine andere, einem Fachmann als sinnvoll erscheinende Art und Weise abzugeben, wie beispielsweise mittels einer Anzeige in einem Display, mittels eines Lautsprechers o. dgl.

Die Vorrichtung 10a umfasst zudem zumindest eine Vorratseinheit 16a zu einer Bevorratung von zumindest einem Bestandteil zur Herstellung der individuell angepassten Verabreichungs- und/oder Betriebshilfsstoffeinheit. Hierbei kann der Bestandteil zur Herstellung der individuell angepassten Verabreichungs- und/oder Betriebshilfsstoffeinheit flüssig, fest und/oder gasförmig ausgebildet sein. Somit kann die Vorratseinheit 16a als Flüssigstoffvorratseinheit, als Feststoffvorratseinheit und/oder als Gasstoffvorratseinheit ausgebildet sein. Die Vorratseinheit 16a weist zumindest zwei getrennte Kammern 18a, 20a zu einer Bevorratung von zumindest zwei unterschiedlichen Bestandteilen der individuell angepassten Verabreichungs- und/oder Betriebshilfsstoffeinheit auf. Bevorzugt weist die Vorratseinheit 16a eine von zwei abweichende Anzahl an getrennten Kammern 18a, 20a zu einer Bevorratung von zumindest zwei unterschiedlichen Bestandteilen der individuell angepassten Verabreichungs- und/oder Betriebshilfsstoffeinheit auf. Die Kammern 18a, 20a sind hierbei als Vorratsbehälter ausgebildet. Die Vorratsbehälter sind austauschbar an der Vorrichtung 10a anordenbar. Somit sind in Abhängigkeit einer Zusammensetzung der gewünschten Verabreichungs- und/oder Betriebshilfsstoffeinheit verschiedene Vorratsbehälter mit unterschiedlichen Bestandteilen der Verabreichungs- und/oder Betriebshilfsstoffeinheit an der Vorrichtung 10a anordenbar. Die Vorratseinheit 16a weist hierzu eine von zwei abweichende Vielzahl an Vorratsbehälteraufnahmeelemente (hier nicht näher dargestellt) auf.

Des Weiteren umfasst die Vorrichtung 10a zumindest eine Dosiereinheit 22a zu einer Dosierung von zumindest einem Bestandteil der individuell angepassten Verabreichungs- und/oder Betriebshilfsstoffeinheit und/oder zu einer Ausgabedosierung der individuell angepassten Verabreichungs- und/oder Betriebshilfsstoffeinheit. Die Dosiereinheit 22a umfasst hierzu zumindest ein Dosierelement (hier nicht näher dargestellt), das mit zumindest einer der Kammern 18a, 20a dosiertechnisch verbunden ist. Das Dosierelement ist somit dazu vorgesehen, eine Abgabe von zumindest einem Bestandteil zur Herstellung der individuell angepassten Verabreichungs- und/oder Betriebshilfsstoffeinheit aus einer der Kammern 18a, 20a zu steuern und/oder zu regeln. Insgesamt weist die Dosiereinheit 22a eine Vielzahl an Dosierelementen auf, die einer Anzahl an vorhandenen Kammern 18a, 20a der Vorratseinheit 16a entspricht. Ferner umfasst die Dosiereinheit 22a zumindest ein Ausgabedosierelement 46a, das dazu vorgesehen ist, eine Ausgabemenge der Verabreichungs- und/oder Betriebshilfsstoffeinheit zu dosieren. Das Ausgabedosierelement 46a kann hierbei als Ausgabedosierventil ausgebildet sein. Es ist jedoch auch denkbar, dass das Ausgabedosierelement 46a eine andere, einem Fachmann als sinnvoll erscheinende Ausgestaltung aufweist, wie beispielsweise eine Ausgestaltung als Ausgabedosierdüse, als Ausgabedosierschieber o. dgl. Die Dosiereinheit 22a kann dazu vorgesehen sein, eine Dosierung von zumindest einem Bestandteil der individuell angepassten Verabreichungs- und/oder Betriebshilfsstoffeinheit und/oder eine Ausgabedosierung der Verabreichungs- und/oder Betriebshilfsstoffeinheit in Abhängigkeit von einem Gewicht der einzelnen Bestandteile, von einem Gewicht des Vorratsbehälters, von einer Anzahl von Bestandteilmengeneinheiten, von einer Durchflussmessung, von einer Ventilsteuerung, von einer Veränderung eines Durchschnittes, von einer Zeit, von einer optischen Komponente, von einer Vermessungskenngröße o. dgl. vorzunehmen.

Die Vorrichtung 10a umfasst zumindest eine Mischeinheit 24a zu einer Mischung von Bestandteilen der individuell angepassten Verabreichungs- und/oder Betriebshilfsstoffeinheit. Die Mischeinheit 24a ist hierbei mit der Dosiereinheit 22a mittels eines Bestandteilzuführkanals (hier nicht näher dargestellt) verbunden. Die Mischeinheit 24a ist als automatische Mischeinheit ausgebildet, die dazu vorgesehen ist, die in Abhängigkeit von der mittels der Rezeptur- und/oder Dosierungsgenerierungseinheit 12a generierten Rezeptur und/oder Dosierung durch die Dosiereinheit 22a dosierten Bestandteile der Verabreichungs- und/oder Betriebshilfsstoffeinheit automatisch zu vermengen. Nach einer Vermengung der Bestandteile der Verabreichungs- und/oder Betriebshilfsstoffeinheit ist es denkbar, dass die vermengten Bestandteile zu einer Fertigstellung einer weiteren Einheit der Vorrichtung 10a, wie beispielsweise einer Presseinheit, einer Heizeinheit, einer Kühleinheit o. dgl., zuführbar sind.

Des Weiteren umfasst die Vorrichtung 10a zumindest eine Verpackungserzeugungseinheit 40a, die dazu vorgesehen ist, eine Verpackung der mittels der Vorrichtung 10a hergestellten individuell angepassten Verabreichungs- und/oder Betriebshilfsstoffeinheit herzustellen. Mittels der Verpackungserzeugungseinheit 40a ist die Verabreichungs- und/oder Betriebshilfsstoffeinheit direkt nach einer Herstellung verpackbar. Die Verpackungserzeugungseinheit 40a kann zusätzlich dazu vorgesehen sein, eine Umverpackung der mittels der Vorrichtung 10a hergestellten individuell angepassten Verabreichungs- und/oder Betriebshilfsstoffeinheit herzustellen.

Ein Verfahren zu einem Betrieb der Vorrichtung 10a weist zumindest einen Verfahrensschritt auf, der zumindest eine individuelle Generierung einer Rezeptur und/oder Dosierung zu einer Herstellung von zumindest einer individuell angepassten Verabreichungs- und/oder Betriebshilfsstoffeinheit und/oder zu einer Verabreichung von zumindest einer individuell angepassten Verabreichungseinheit aufweist. Hierzu ist die zumindest eine individuelle Kenngröße mittels der Eingabeeinheit 26a eingebbar und/oder mittels der Sensoreinheit 30a erfassbar. Die individuelle Kenngröße ist beispielsweise als Puls, als Blutdruck, als Herzrhythmus, als Blutbild, als Zahnzustandskenngröße, als Hautverträglichkeitskenngröße, als Leistungs-EKG-Kenngröße, als Schmierstoffviskosität, als Bauteilleimartverbindungskenngröße o. dgl. ausgebildet. Die Generierung einer Rezeptur und/oder Dosierung ist somit mittels einer manuellen Eingabe von der zumindest einen individuellen Kenngröße durchführbar. Alternativ oder zusätzlich erfolgt eine Erfassung der zumindest einen individuellen Kenngröße zu einer Generierung einer Rezeptur und/oder Dosierung zumindest teilweise automatisch. Die Rezeptur- und/oder Dosierungsgenerierungseinheit 12a generiert hierbei eine Rezeptur und/oder eine Dosierung der Verabreichungs- und/oder Betriebshilfsstoffeinheit in Abhängigkeit von der zumindest einen individuellen Kenngröße. Die generierte Rezeptur und/oder eine Dosierung der Verabreichungs- und/oder Betriebshilfsstoffeinheit kann über die Eingabeeinheit 26a, über die Sensoreinheit 30a und/oder über die Kommunikationseinheit 32a validiert, geändert und/oder durch weitere Angaben, wie beispielsweise Blutwerte o. dgl., ergänzt werden. Die Herstellung der individuell angepassten Verabreichungs- und/oder Betriebshilfsstoffeinheit und/oder die Verabreichung der individuell angepassten Verabreichungseinheit zumindest in Abhängigkeit von der zumindest einen individuellen Kenngröße erfolgen/erfolgt zumindest teilweise automatisch. Hierbei erfolgt eine Ausgabe der hergestellten Verabreichungs- und/oder Betriebshilfsstoffeinheit mittels des Ausgabedosierelements der Dosiereinheit 22a.

Hierbei ist es denkbar, dass die individuell angepasste Verabreichungs- und/oder Betriebshilfsstoffeinheit mittels der Vorrichtung 10a zusammen mit einem individuellen Beipackzettel und eine dazugehörige Verpackung produzierbar ist. Es ist jedoch auch denkbar, dass die Rezeptur und/oder die Dosierung mittels eines Implantats oder ein anderes, einem Fachmann als sinnvoll erscheinenden Trägermediums, wie beispielsweise mittels eines Pflasters o. dgl., verabreichbar ist. Das Implantat oder ein anderes, einem Fachmann als sinnvoll erscheinendes Trägermedium ist von einem Bediener wieder befüllbar ausgebildet. Zudem ist es denkbar, dass die generierte Rezeptur und/oder eine Dosierung der Verabreichungs- und/oder Betriebshilfsstoffeinheit zu einer Überprüfung und/oder zu einer Herstellung an einen Arzt und/oder Apotheke übermittebar sind/ist. Somit erfolgt in zumindest einem Verfahrensschritt ein Abgleich und/oder eine Überprüfung der individuell abgestimmten Rezeptur und/oder Dosierung. Hierbei sind die Rezeptur und/oder Dosierung mittels der Kommunikationseinheit 32a an eine externe Einheit 34a des Arztes und/oder des Apothekers übertragbar. Somit erfolgt der Abgleich und/oder die Überprüfung der individuell abgestimmten Rezeptur und/oder Dosierung infolge einer elektronischen Datenübertragung. Die Herstellung von zumindest einer individuell angepassten Verabreichungs- und/oder Betriebshilfsstoffeinheit und/oder die Verabreichung von zumindest einer individuell angepassten Verabreichungseinheit zumindest in Abhängigkeit von der zumindest einen individuellen Kenngröße erfolgen/erfolgt zumindest teilweise automatisch mittels der Vorrichtung 10a.

In Figuren 2 bis 7 sind weitere Ausführungsbeispiele der Erfindung gezeigt. Die nachfolgenden Beschreibungen und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung der anderen Ausführungsbeispiele verwiesen werden kann. Zur Unterscheidung der Ausführungsbeispiele ist der Buchstabe a den Bezugszeichen des Ausführungsbeispiels in der Figur 1 nachgestellt. In den Ausführungsbeispielen der Figuren 2 bis 7 ist der Buchstabe a durch die Buchstaben b bis f ersetzt.

Figur 2 zeigt eine alternative Vorrichtung 10b zu einer Herstellung von zumindest einer individuell angepassten Verabreichungs- und/oder Betriebshilfsstoffeinheit und/oder zu einer Verabreichung von zumindest einer individuell angepassten Verabreichungseinheit in Abhängigkeit von zumindest einer individuellen Kenngröße. Im Unterschied zu der in der Figur 1 dargestellten Vorrichtung 10a ist die in der Figur 2 dargestellte Vorrichtung 10b als mobile Vorrichtung ausgebildet, die von einem Individuum am Körper tragbar ist. Hierbei kann die Vorrichtung 10b als Pflaster, als Medikamentenverabreichungsvorrichtung, wie beispielsweise als Insulinpumpenvorrichtung o. dgl. ausgebildet sein. Bei einer Ausgestaltung der Vorrichtung 10b als Pflaster ist es denkbar, dass die Vorrichtung 10b als Nanotechnologievorrichtung ausgebildet ist. Hierbei ist es denkbar, dass die als Nanotechnologievorrichtung ausgebildete Vorrichtung 10b dazu vorgesehen ist, Verabreichungseinheiten über eine dünne Heizschicht in eine Haut eines Trägers der Vorrichtung 10a abzugeben, wobei ein Hitzesensorelement der Vorrichtung verhindert, dass die Haut dabei überhitzt wird.

Zudem weist die in der Figur 2 dargestellte Vorrichtung 10b Im Unterschied zu der in der Figur 1 dargestellten Vorrichtung 10a zumindest eine Injektionseinheit 42a zu einer Injektion zumindest einer individuell angepassten Verabreichungseinheit auf. Hierzu umfasst die Injektionseinheit 42a zumindest ein Injektionselement (hier nicht näher dargestellt) auf, das zu einer Injektion der mittels der Vorrichtung 10b hergestellten Verabreichungseinheit vorgesehen ist. Das Injektionselement ist hierbei als Injektionsnadel ausgebildet. Es ist jedoch auch denkbar, dass das Injektionselement eine andere, einem Fachmann als sinnvoll erscheinende Ausgestaltung aufweist. Des Weiteren ist eine Eingabeeinheit 26b der Vorrichtung 10b ein Teil einer externen Einheit 34b. Die externe Einheit 34b ist mittels einer Kommunikationseinheit 32b der Vorrichtung 10b zu einem elektronischen Datenaustausch mit der Vorrichtung 10b verbunden. Eine Umgebungskenngrößenerfassungseinheit 36b der Vorrichtung 10b kann ebenfalls in der externen Einheit 34b angeordnet sein und mittels der Kommunikationseinheit 32b elektronische Daten mit der Vorrichtung 10b austauschen. Hinsichtlich weiterer Merkmale und Funktionen der in der Figur 2 dargestellten Vorrichtung 10b darf auf die Beschreibung der in der Figur 1 dargestellten Vorrichtung 10a verwiesen werden.

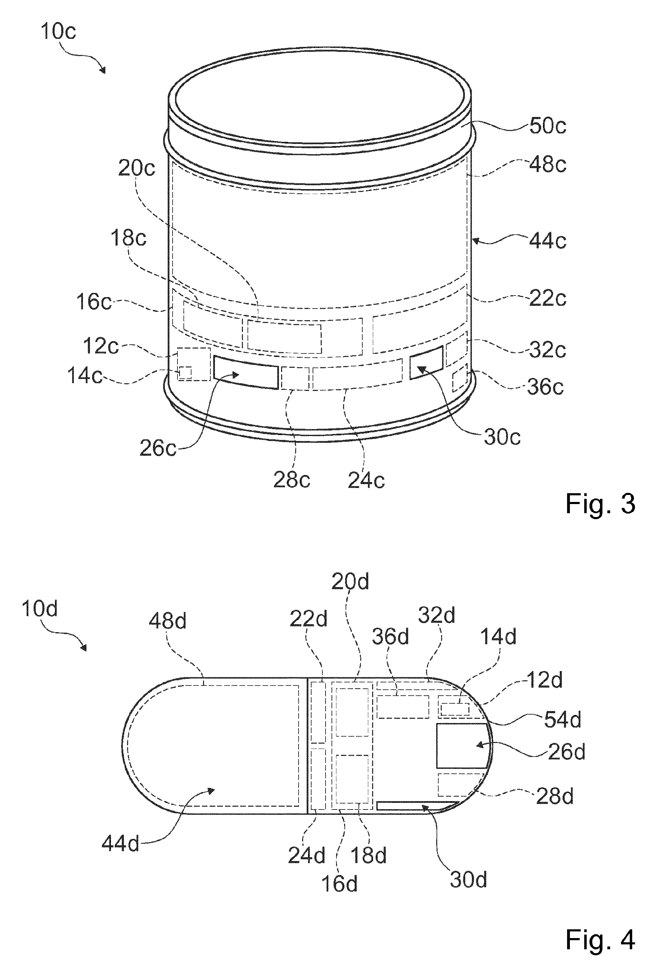

Figur 3 zeigt eine weitere, alternative Vorrichtung 10c zu einer Herstellung von zumindest einer individuell angepassten Verabreichungs- und/oder Betriebshilfsstoffeinheit und/oder zu einer Verabreichung von zumindest einer individuell angepassten Verabreichungseinheit in Abhängigkeit von zumindest einer individuellen Kenngröße. Im Unterschied zu der in der Figur 1 dargestellten Vorrichtung 10a ist die in der Figur 3 dargestellte Vorrichtung 10c dosenförmig ausgebildet. Hierbei weist die Vorrichtung 10c eine als Dose ausgebildete Gehäuseeinheit 44c auf. Die Gehäuseeinheit 44c ist dazu vorgesehen, weitere Einheiten (Rezeptur- und/oder Dosierungsgenerierungseinheit 12c, Vorratseinheit 16c, Dosiereinheit 22c, Mischeinheit 24c, Eingabeeinheit 26c, Auswerteeinheit 28c, Sensoreinheit 30c, Kommunikationseinheit 32c und/oder Umgebungskenngrößenerfassungseinheit 36c) der Vorrichtung 10c aufzunehmen und/oder zu lagern.

Die Vorrichtung 10c umfasst zumindest eine Vorratseinheit 16c zu einer Bevorratung von zumindest einem Bestandteil zur Herstellung der individuell angepassten Verabreichungs- und/oder Betriebshilfsstoffeinheit. Die Vorratseinheit 16c weist zumindest zwei getrennte Kammern 18c, 20c zu einer Bevorratung von zumindest zwei unterschiedlichen Bestandteilen der individuell angepassten Verabreichungs- und/oder Betriebshilfsstoffeinheit auf. Ferner umfasst die Vorrichtung 10c zumindest eine Dosiereinheit 22c zu einer Dosierung von zumindest einem Bestandteil der individuell angepassten Verabreichungs- und/oder Betriebshilfsstoffeinheit und/oder zu einer Ausgabedosierung der individuell angepassten Verabreichungs- und/oder Betriebshilfsstoffeinheit. Zudem umfasst die Vorrichtung 10c zumindest eine Mischeinheit 24c zu einer Mischung von Bestandteilen der individuell angepassten Verabreichungs- und/oder Betriebshilfsstoffeinheit. Die Vorratseinheit 16c, die Dosiereinheit 22c und die Mischeinheit 24c sind vorzugsweise in einem bodennahen Bereich der als Dose ausgebildeten Gehäuseeinheit 44c angeordnet. Zudem weist die Gehäuseeinheit 44c zumindest eine Aufnahmekammer 48c auf, die zu einer Aufnahme von einer hergestellten Verabreichungs- und/oder Betriebshilfsstoffeinheit vorgesehen ist. Die Aufnahmekammer 48c ist mittels eines Ausgabedosierelements (hier nicht näher dargestellt) der Dosiereinheit 22c befüllbar. Zudem umfasst die Gehäuseeinheit 44c ein Verschlusselement 50c zu einem Verschluss der Aufnahmekammer 48c. Ein Bediener der Vorrichtung 10c kann die hergestellte Verabreichungs- und/oder Betriebshilfsstoffeinheit zu einer Verabreichung aus der Aufnahmekammer 48c entnehmen. Hierbei kann die Verabreichungs- und/oder Betriebshilfsstoffeinheit als Tablette, als Flüssigkeit, als Paste, als Creme, als Pulver o. dgl. ausgebildet sein. Hinsichtlich weiterer Merkmale und Funktionen der in der Figur 3 dargestellten Vorrichtung 10c darf auf die Beschreibung der in der Figur 1 dargestellten Vorrichtung 10a verwiesen werden.

Figur 4 zeigt eine weitere, alternative Vorrichtung 10d zu einer Herstellung von zumindest einer individuell angepassten Verabreichungs- und/oder Betriebshilfsstoffeinheit und/oder zu einer Verabreichung von zumindest einer individuell angepassten Verabreichungseinheit in Abhängigkeit von zumindest einer individuellen Kenngröße. Im Unterschied zu der in der Figur 1 dargestellten Vorrichtung 10a ist die in der Figur 4 dargestellte Vorrichtung 10d kapselförmig ausgebildet. Hierbei weist die Vorrichtung 10d eine als Kapsel ausgebildete Gehäuseeinheit 44d auf. Die Gehäuseeinheit 44d ist dazu vorgesehen, weitere Einheiten (Rezeptur- und/oder Dosierungsgenerierungseinheit 12d, Vorratseinheit 16d, Dosiereinheit 22d, Mischeinheit 24d, Eingabeeinheit 26d, Auswerteeinheit 28d, Sensoreinheit 30d, Kommunikationseinheit 32d und/oder Umgebungskenngrößenerfassungseinheit 36d) der Vorrichtung 10d aufzunehmen und/oder zu lagern.

Die Vorrichtung 10d umfasst zumindest eine Vorratseinheit 16c zu einer Bevorratung von zumindest einem Bestandteil zur Herstellung der individuell angepassten Verabreichungs- und/oder Betriebshilfsstoffeinheit. Die Vorratseinheit 16d weist zumindest zwei getrennte Kammern 18d, 20d zu einer Bevorratung von zumindest zwei unterschiedlichen Bestandteilen der individuell angepassten Verabreichungs- und/oder Betriebshilfsstoffeinheiten auf. Ferner umfasst die Vorrichtung 10d zumindest eine Dosiereinheit 22d zu einer Dosierung von zumindest einem Bestandteil der individuell angepassten Verabreichungs- und/oder Betriebshilfsstoffeinheit und/oder zu einer Ausgabedosierung der individuell angepassten Verabreichungs- und/oder Betriebshilfsstoffeinheit. Zudem umfasst die Vorrichtung 10d zumindest eine Mischeinheit 24d zu einer Mischung von Bestandteilen der individuell angepassten Verabreichungs- und/oder Betriebshilfsstoffeinheit. Zudem weist die Gehäuseeinheit 44d zumindest eine Aufnahmekammer 48d auf, die zu einer Aufnahme von einer hergestellten Verabreichungs- und/oder Betriebshilfsstoffeinheit vorgesehen ist. Die Aufnahmekammer 48d ist mittels eines Ausgabedosierelements (hier nicht näher dargestellt) der Dosiereinheit 22d befüllbar. Die Aufnahmekammer 48d ist nach einer Befüllung mit einem Verschlusselement 50d der Gehäuseeinheit 44d verschließbar. Zudem ist die Aufnahmekammer 48d abnehmbar an einem Grundkörper 54d der Gehäuseeinheit 44d angeordnet. Die Aufnahmekammer 48d ist hierbei aus einem leicht verdaulichen Material gebildet. Somit kann die befüllte Aufnahmekammer 48d, insbesondere nach einer Befüllung mit einer Verabreichungs- und/oder Betriebshilfsstoffeinheit, oral verabreicht werden. Es ist jedoch auch denkbar, dass die Aufnahmekammer 48d eine andere, einem Fachmann als sinnvoll erscheinende Ausgestaltung aufweist und auf eine andere Art und Weise verabreicht werden kann. Die Aufnahmekammer 48d ist somit austauschbar und/oder einmalverwendbar ausgebildet. Hinsichtlich weiterer Merkmale und Funktionen der in der Figur 4 dargestellten Vorrichtung 10d darf auf die Beschreibung der in der Figur 1 dargestellten Vorrichtung 10a verwiesen werden.

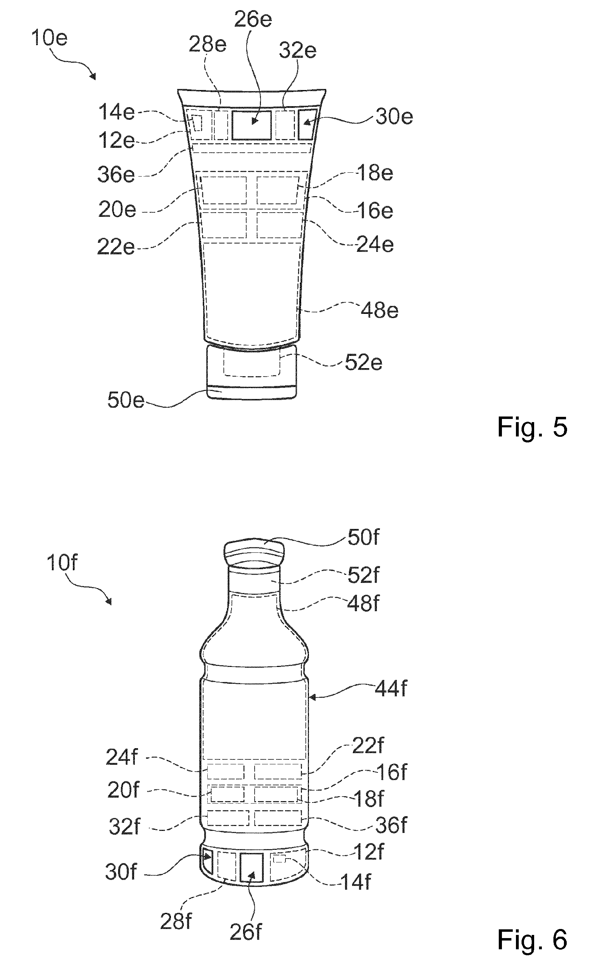

Figur 5 zeigt eine weitere, alternative Vorrichtung 10e zu einer Herstellung von zumindest einer individuell angepassten Verabreichungs- und/oder Betriebshilfsstoffeinheit und/oder zu einer Verabreichung von zumindest einer individuell angepassten Verabreichungseinheit in Abhängigkeit von zumindest einer individuellen Kenngröße. Im Unterschied zu der in der Figur 1 dargestellten Vorrichtung 10a ist die in der Figur 5 dargestellte Vorrichtung 10e tubenförmig ausgebildet. Hierbei weist die Vorrichtung 10e eine als Tube ausgebildete Gehäuseeinheit 44e auf. Die Gehäuseeinheit 44e ist dazu vorgesehen, weitere Einheiten (Rezeptur- und/oder Dosierungsgenerierungseinheit 12e, Vorratseinheit 16e, Dosiereinheit 22e, Mischeinheit 24e, Eingabeeinheit 26e, Auswerteeinheit 28e, Sensoreinheit 30e, Kommunikationseinheit 32e und/oder Umgebungskenngrößenerfassungseinheit 36e) der Vorrichtung 10e aufzunehmen und/oder zu lagern.

Die Vorrichtung 10e umfasst zumindest eine Vorratseinheit 16e zu einer Bevorratung von zumindest einem Bestandteil zur Herstellung der individuell angepassten Verabreichungs- und/oder Betriebshilfsstoffeinheit. Die Vorratseinheit 16e weist zumindest zwei getrennte Kammern 18e, 20e zu einer Bevorratung von zumindest zwei unterschiedlichen Bestandteilen der individuell angepassten Verabreichungs- und/oder Betriebshilfsstoffeinheit auf. Ferner umfasst die Vorrichtung 10e zumindest eine Dosiereinheit 22e zu einer Dosierung von zumindest einem Bestandteil der individuell angepassten Verabreichungs- und/oder Betriebshilfsstoffeinheit und/oder zu einer Ausgabedosierung der individuell angepassten Verabreichungs- und/oder Betriebshilfsstoffeinheit. Zudem umfasst die Vorrichtung 10e zumindest eine Mischeinheit 24e zu einer Mischung von Bestandteilen der individuell angepassten Verabreichungs- und/oder Betriebshilfsstoffeinheit. Zudem weist die Gehäuseeinheit 44e zumindest eine Aufnahmekammer 48e auf, die zu einer Aufnahme von einer hergestellten Verabreichungs- und/oder Betriebshilfsstoffeinheit vorgesehen ist. Die Aufnahmekammer 48e ist hierbei zwischen einem Ausgabedosierelement 52e und der Vorratseinheit 16e der Dosiereinheit 22e angeordnet. Somit ist das Ausgabedosierelement 52e dazu vorgesehen, die in der Aufnahmekammer 48e anordenbare Verabreichungs- und/oder Betriebshilfsstoffeinheit in Abhängigkeit von einer mittels einer Rezeptur- und/oder Dosierungsgenerierungseinheit 12e der Vorrichtung 10e generierten Dosierung abzugeben. Es ist jedoch auch denkbar, dass das Ausgabedosierelement 52e direkt mit der Mischeinheit 24e verbunden ist, um die Verabreichungs- und/oder Betriebshilfsstoffeinheit direkt nach einer Herstellung abzugeben. Zudem ist es auch denkbar, dass die Vorrichtung 10e mittels austauschbaren Verabreichungseinheitskartuschen bestückbar ist und lediglich eine Menge in Abhängigkeit von einer mittels einer Rezeptur- und/oder Dosierungsgenerierungseinheit 12e der Vorrichtung 10e generierten Dosierung abgebbar ist. Hinsichtlich weiterer Merkmale und Funktionen der in der Figur 5 dargestellten Vorrichtung 10e darf auf die Beschreibung der in der Figur 1 dargestellten Vorrichtung 10a verwiesen werden.

Figur 6 zeigt eine weitere, alternative Vorrichtung 10f zu einer Herstellung von zumindest einer individuell angepassten Verabreichungs- und/oder Betriebshilfsstoffeinheit und/oder zu einer Verabreichung von zumindest einer individuell angepassten Verabreichungseinheit in Abhängigkeit von zumindest einer individuellen Kenngröße. Im Unterschied zu der in der Figur 1 dargestellten Vorrichtung 10a ist die in der Figur 6 dargestellte Vorrichtung 10f flaschenförmig ausgebildet. Hierbei weist die Vorrichtung 10f eine als Dose ausgebildete Gehäuseeinheit 44f auf. Die Gehäuseeinheit 44f ist dazu vorgesehen, weitere Einheiten (Rezeptur- und/oder Dosierungsgenerierungseinheit 12f, Vorratseinheit 16f, Dosiereinheit 22f, Mischeinheit 24f, Eingabeeinheit 26f, Auswerteeinheit 28f, Sensoreinheit 30f, Kommunikationseinheit 32f und/oder Umgebungskenngrößenerfassungseinheit 36f) der Vorrichtung 10f aufzunehmen und/oder zu lagern.

Die Vorrichtung 10f umfasst zumindest eine Vorratseinheit 16f zu einer Bevorratung von zumindest einem Bestandteil zur Herstellung der individuell angepassten Verabreichungs- und/oder Betriebshilfsstoffeinheit. Die Vorratseinheit 16f weist zumindest zwei getrennte Kammern 18f, 20f zu einer Bevorratung von zumindest zwei unterschiedlichen Bestandteilen der individuell angepassten Verabreichungs- und/oder Betriebshilfsstoffeinheit auf. Ferner umfasst die Vorrichtung 10f zumindest eine Dosiereinheit 22f zu einer Dosierung von zumindest einem Bestandteil der individuell angepassten Verabreichungs- und/oder Betriebshilfsstoffeinheit und/oder zu einer Ausgabedosierung der individuell angepassten Verabreichungs- und/oder Betriebshilfsstoffeinheit. Zudem umfasst die Vorrichtung 10f zumindest eine Mischeinheit 24f zu einer Mischung von Bestandteilen der individuell angepassten Verabreichungs- und/oder Betriebshilfsstoffeinheit. Die Vorratseinheit 16f, die Dosiereinheit 22f und die Mischeinheit 24f sind vorzugsweise in einem bodennahen Bereich der als Flasche ausgebildeten Gehäuseeinheit 44f angeordnet. Zudem weist die Gehäuseeinheit 44f zumindest eine Aufnahmekammer 48f auf, die zu einer Aufnahme von einer hergestellten Verabreichungs- und/oder Betriebshilfsstoffeinheit vorgesehen ist. Die Aufnahmekammer 48f ist mittels eines Ausgabedosierelements (hier nicht näher dargestellt) der Dosiereinheit 22f befüllbar. Zudem umfasst die Gehäuseeinheit 44f ein Verschlusselement 50f zu einem Verschluss der Aufnahmekammer 48f. Ein Bediener der Vorrichtung 10f kann die hergestellte Verabreichungs- und/oder Betriebshilfsstoffeinheit zu einer Verabreichung aus der Aufnahmekammer 48f entnehmen. Hierbei ist die Verabreichungs- und/oder Betriebshilfsstoffeinheit als Flüssigkeit, wie beispielsweise als Nahrungsergänzungsmittel o. dgl., ausgebildet. Hinsichtlich weiterer Merkmale und Funktionen der in der Figur 6 dargestellten Vorrichtung 10f darf auf die Beschreibung der in der Figur 1 dargestellten Vorrichtung 10a verwiesen werden.

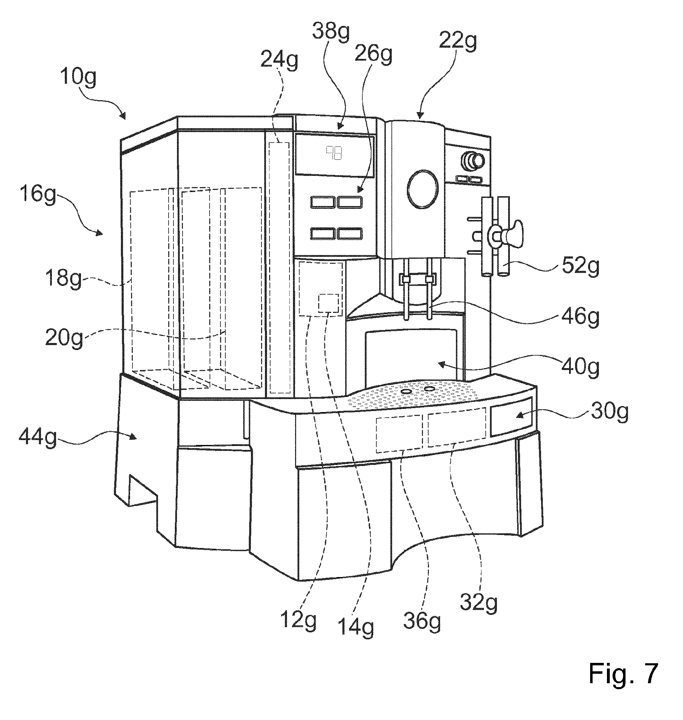

Figur 7 zeigt eine weitere, alternative Vorrichtung 10g zu einer Herstellung von zumindest einer individuell angepassten Verabreichungs- und/oder Betriebshilfsstoffeinheit und/oder zu einer Verabreichung von zumindest einer individuell angepassten Verabreichungseinheit in Abhängigkeit von zumindest einer individuellen Kenngröße. Im Unterschied zu der in der Figur 1 dargestellten Vorrichtung 10a ist die in der Figur 7 dargestellte Vorrichtung 10g dazu vorgesehen, eine als Flüssigkeit und/oder als Brei ausgebildete Verabreichungs- und/oder Betriebshilfsstoffeinheit herzustellen und/oder eine als Flüssigkeit ausgebildete Verabreichungseinheit zu verabreichen. Hierbei weist die Vorrichtung 10g eine getränkemaschinenartig ausgebildete Gehäuseeinheit 44g auf. Die Gehäuseeinheit 44g ist dazu vorgesehen, weitere Einheiten (Rezeptur- und/oder Dosierungsgenerierungseinheit 12g, Vorratseinheit 16g, Dosiereinheit 22g, Mischeinheit 24g, Eingabeeinheit 26g, Auswerteeinheit 28g, Sensoreinheit 30g, Kommunikationseinheit 32g und/oder Umgebungskenngrößenerfassungseinheit 36g) der Vorrichtung 10g aufzunehmen und/oder zu lagern. Hinsichtlich weiterer Merkmale und Funktionen der in der Figur 7 dargestellten Vorrichtung 10g darf auf die Beschreibung der in der Figur 1 dargestellten Vorrichtung 10a verwiesen werden.

## Patentansprüche

1. Vorrichtung zu einer Herstellung von zumindest einer individuell angepassten Verabreichungs- und/oder Betriebshilfsstoffeinheit und/oder zu einer Verabreichung von zumindest einer individuell angepassten Verabreichungseinheit in Abhängigkeit von zumindest einer individuellen Kenngröße.

2. Vorrichtung nach Anspruch 1, **gekennzeichnet durch** zumindest eine Rezeptur- und/oder Dosierungsgenerierungseinheit (12a; 12b; 12c; 12d; 12e; 12f; 12g), die dazu vorgesehen ist, eine Rezeptur und/oder eine Dosierung in Abhängigkeit von der zumindest einen individuellen Kenngröße zu generieren.

3. Vorrichtung nach Anspruch 2, **dadurch gekennzeichnet, dass** die Rezeptur- und/oder Dosierungsgenerierungseinheit (12a; 12b; 12c; 12d; 12e; 12f; 12g) zumindest eine Steuer- und/oder Regeleinheit (14a; 14b; 14c; 14d; 14e; 14f; 14g) umfasst, die zumindest ein Betriebsprogramm aufweist, das in Abhängigkeit einer Eingabe von der zumindest einen individuellen Kenngröße ein Rezeptur- und/oder Dosierungsprofil erstellt.

4. Vorrichtung nach einem der vorhergehenden Ansprüche, **gekennzeichnet durch** zumindest eine Vorratseinheit (16a; 16b; 16c; 16d; 16e; 16f; 16g) zu einer Bevorratung von zumindest einem Bestandteil zur Herstellung der individuell angepassten Verabreichungs- und/oder Betriebshilfsstoffeinheit.

5. Vorrichtung nach Anspruch 4, **dadurch gekennzeichnet, dass** die Vorratseinheit (16a; 16b; 16c; 16d; 16e; 16f; 16g) zumindest zwei getrennte Kammern (18a, 20a; 18b, 20b; 18c, 20c; 18d, 20d; 18e, 20e; 18f, 20f; 18g, 20g) zu einer Bevorratung von zumindest zwei unterschiedlichen Bestandteilen der individuell angepassten Verabreichungs- und/oder Betriebshilfsstoffeinheiten aufweist.

6. Vorrichtung nach einem der vorhergehenden Ansprüche, **gekennzeichnet durch** zumindest eine Dosiereinheit (22a; 22b; 22c; 22d; 22e; 22f; 22g) zu einer Dosierung von zumindest einem Bestandteil der individuell angepassten Verabreichungs- und/oder Betriebshilfsstoffeinheit und/oder zu einer Ausgabedosierung der individuell angepassten Verabreichungs- und/oder Betriebshilfsstoffeinheit.

7. Vorrichtung nach einem der vorhergehenden Ansprüche, **gekennzeichnet durch** zumindest eine Mischeinheit (24a; 24b; 24c; 24d; 24e; 24f; 24g) zu einer Mischung von Bestandteilen der individuell angepassten Verabreichungs- und/oder Betriebshilfsstoffeinheit.

8. Vorrichtung nach einem der vorhergehenden Ansprüche, **gekennzeichnet durch** zumindest eine Eingabeeinheit (26a; 26b; 26c; 26d; 26e; 26f; 26g) zu einer manuellen Eingabe von zumindest einer individuell abgestimmten Rezeptur und/oder Dosierung zur Herstellung der individuell angepassten Verabreichungs- und/oder Betriebshilfsstoffeinheit.

9. Vorrichtung nach einem der vorhergehenden Ansprüche, **gekennzeichnet durch** zumindest eine Auswerteeinheit (28a; 28b; 28c; 28d; 28e; 28f; 28g), die dazu vorgesehen ist, die zumindest eine individuell eingegebene Kenngröße zumindest hinsichtlich einer Generierung einer individuell abgestimmten Rezeptur und/oder Dosierung auszuwerten.

10. Vorrichtung nach einem der vorhergehenden Ansprüche, **gekennzeichnet durch** zumindest eine Sensoreinheit (30a; 30b; 30c; 30d; 30e; 30f; 30g), die dazu vorgesehen ist, die zumindest eine individuelle Kenngröße zu ermitteln, die zu einer Generierung einer individuell abgestimmten Rezeptur und/oder Dosierung nutzbar ist.

11. Vorrichtung nach einem der vorhergehenden Ansprüche, **gekennzeichnet durch** zumindest eine Kommunikationseinheit (32a; 32b; 32c; 32d; 32e; 32f; 32g), die dazu vorgesehen ist, mit zumindest einer externen Einheit (34a; 34b) elektronische Daten auszutauschen.

12. Vorrichtung nach einem der vorhergehenden Ansprüche, **gekennzeichnet durch** zumindest eine Umgebungskenngrößenerfassungseinheit (36a; 36b; 36c; 36d; 36e; 36f; 36g), die dazu vorgesehen ist, zumindest eine Umgebungskenngröße zu einer Generierung einer individuell abgestimmten Rezeptur und/oder Dosierung zu erfassen.

13. Vorrichtung nach einem der vorhergehenden Ansprüche, **gekennzeichnet durch** zumindest eine Rezepturausgabeeinheit (38a; 38b; 38g), die dazu vorgesehen ist, zumindest eine individuelle abgestimmte Rezeptur und/oder Dosierung auszugeben.

14. Vorrichtung nach einem der vorhergehenden Ansprüche, **gekennzeichnet durch** zumindest eine Verpackungserzeugungseinheit (40a; 40g), die dazu vorgesehen ist, eine Verpackung der individuell angepassten Verabreichungs- und/oder Betriebshilfsstoffeinheit herzustellen.

15. Vorrichtung nach einem der vorhergehenden Ansprüche, **gekennzeichnet durch** zumindest eine Injektionseinheit (42b) zu einer Injektion zumindest einer individuell angepassten Verabreichungseinheit.

16. Rezeptur- und/oder Dosierungsgenerierungseinheit (12a; 12b; 12c; 12d; 12e;
12f; 12g) einer Vorrichtung nach einem der vorhergehenden Ansprüche.

17. Verfahren zu einem Betrieb der Vorrichtung nach einem der Ansprüche 1 bis 15, **gekennzeichnet durch** eine individuelle Generierung einer Rezeptur und/oder Dosierung zu einer Herstellung von einer individuell angepassten Verabreichungs- und/oder Betriebshilfsstoffeinheit und/oder zu einer Verabreichung von individuell angepassten Verabreichungseinheit.

18. Verfahren nach Anspruch 17, **dadurch gekennzeichnet, dass** die Generierung einer Rezeptur und/oder Dosierung mittels einer manuellen Eingabe von der zumindest einen individuellen Kenngröße durchführbar ist.

19. Verfahren nach Anspruch 17 oder 18, **dadurch gekennzeichnet, dass** eine Erfassung der zumindest einen individuellen Kenngröße zu einer Generierung einer Rezeptur und/oder Dosierung zumindest teilweise automatisch erfolgt.

20. Verfahren nach einem der Ansprüche 17 bis 19, **dadurch gekennzeichnet, dass** die Herstellung der individuell angepassten Verabreichungs- und/oder Betriebshilfsstoffeinheit und/oder die Verabreichung der individuell angepassten Verabreichungseinheit zumindest in Abhängigkeit von der zumindest einen individuellen Kenngröße zumindest teilweise automatisch erfolgen/erfolgt.

21. Verfahren nach einem der Ansprüche 17 bis 20, **dadurch gekennzeichnet, dass** in zumindest einem Verfahrensschritt ein Abgleich und/oder eine Überprüfung der individuell abgestimmten Rezeptur und/oder Dosierung erfolgt.

22. Verfahren nach Anspruch 21, **dadurch gekennzeichnet, dass** der Abgleich und/oder die Überprüfung der individuell abgestimmten Rezeptur und/oder Dosierung infolge einer elektronischen Datenübertragung erfolgt.
